Europäisches Patentamt

European Patent Office

Office européen des brevets

(11) Veröffentlichungsnummer: **0 145 892**
**A1**

## (12) EUROPÄISCHE PATENTANMELDUNG

(21) Anmeldenummer: 84112720.2

(22) Anmeldetag: 22.10.84

(51) Int. Cl.⁴: **A 61 B 6/04,** H 05 B 3/36,
A 61 G 13/00

(30) Priorität: 03.11.83 DE 3339781

(43) Veröffentlichungstag der Anmeldung: 26.06.85
Patentblatt 85/26

(84) Benannte Vertragsstaaten: **DE FR**

(71) Anmelder: **Siemens Aktiengesellschaft, Berlin und
München Wittelsbacherplatz 2, D-8000 München 2 (DE)**

(72) Erfinder: **Vogel, Georg, Am Elchengarten 9,
D-8521 Buckenhof (DE)**

(54) **Rontgendiagnostikgerät.**

(57) Die Erfindung bezieht sich auf ein Röntgendiagnostikgerät mit einer Patientenlagerungsplatte (4), die aus Kohlenstoffasermaterial besteht. An der Patientenlagerungsplatte (4) ist eine Heizstromquelle (19) angeschlossen, die
durch das Kohlenstoffasermaterial einen Heizstrom zur Beheizung der Lagerfläche (18) schickt.

EP 0 145 892 A1

Siemens Aktiengesellschaft       Unser Zeichen
Berlin und München               VPA **83 P 3378 E**

## Röntgendiagnostikgerät

Die Erfindung betrifft ein Röntgendiagnostikgerät mit einer Patientenlagerungsplatte, die aus Kohlenstofffasermaterial besteht.

Bei einem Röntgendiagnostikgerät dieser Art kann die Patientenlagerungsplatte bei hoher Festigkeit relativ dünn und leicht ausgebildet werden. Ferner besitzt sie eine nur geringe Strahlenabsorption. Aufgrund der geringen Materialstärke ist auch die Streustrahlung klein.

Die bekannten Patientenlagerungsplatten, also nicht nur diejenigen, die aus Kohlenstoffasermaterial bestehen, fühlen sich für einen daraufgelegten Patienten, insbesondere dann, wenn er für eine Röntgenuntersuchung mit der bloßen Haut mit der Patientenlagerungsplatte in Berührung kommt, häufig kalt an, was sehr störend ist.

Der Erfindung liegt die Aufgabe zugrunde, ein Röntgendiagnostikgerät der eingangs genannten Art so auszubilden, daß für den Patienten beim Berühren der Patientenlagerungsplatte mit der bloßen Haut kein Kältegefühl auftritt.

Diese Aufgabe ist erfindungsgemäß dadurch gelöst, daß an der Patientenlagerungsplatte eine Heizstromquelle angeschlossen ist, die durch das Kohlenstoffasermaterial einen Heizstrom zur Beheizung der Lagerfläche schickt. Die Erfindung geht demgemäß davon aus, daß eine Patientenlagerungsplatte aus Kohlenstoffasermaterial ein elektrischer Widerstandsleiter ist. Bei

Tp 2 Ler / 25.10.1983

geeigneter Bemessung der Patientenlagerungsplatte und
der Heizstromquelle ist es demgemäß möglich eine Aufheizung auf Körpertemperatur zu erzielen.

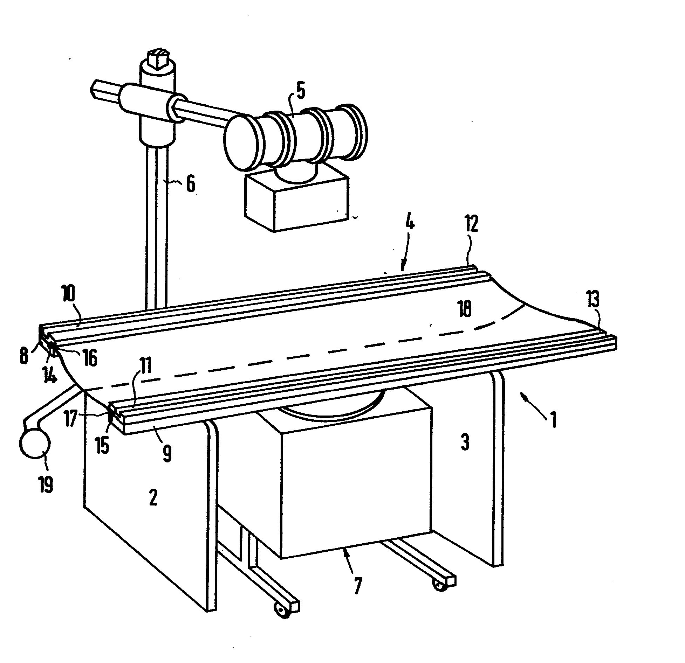

Die Erfindung ist nachfolgend anhand eines in der Zeichnung dargestellten Ausführungsbeispieles näher erläutert.

Die Zeichnung zeigt ein Röntgendiagnostikgerät, bei dem
über einer Patientenlagerstatt 1 mit einer auf zwei Fußgestellen 2, 3 ruhenden zweidimensional verschiebbaren
Patientenlagerungsplatte 4 eine Röntgenröhre 5 an einem
längs der Patientenlagerstatt 1 verschiebbaren Stativ 6
gehaltert ist. Als Aufnahmeeinrichtung ist bei diesem
Röntgendiagnostikgerät ein in einem unter die Patientenlagerungsplatte 4 verschiebbaren Gehäuse 7 untergebrachter Röntgenbildverstärker vorgesehen. Die Patientenlagerungsplatte 4 weist zwei Längsholme 8, 9 auf, auf denen
zur Befestigung von Zubehörteilen je eine mit einer Nut
10, 11 versehene Leiste 12, 13 geschraubt ist. An den
aufeinanderliegenden Seiten der Längsholme 8, 9 und der
zugehörigen Nutenleisten 12, 13 verläuft jeweils in
Längsrichtung eine Kerbe 14, 15, in die eine wulstartige
Verdickung 16, 17 der Ränder der Lagerfläche 18 der
Patientenlagerungsplatte 4 eingespannt ist. Die ganz aus
mit Kohlenstoffasermaterial verstärktem Kunstharz
hergestellte Lagerfläche 18 hängt zwischen den beiden
Längsholmen 8, 9 leicht durch.

Zur Beheizung der Lagerfläche 18 ist diese an ihren
beiden Enden an einer Heizstromquelle 19 angeschlossen,
die durch das Kohlenstoffasermaterial einen Heizstrom
schickt, der die Temperatur der Lagerfläche 18 auf etwa Körpertemperatur erhöht.

1 Patentanspruch
1 Figur

Patentanspruch

Röntgendiagnostikgerät mit einer Patientenlagerungsplatte (4), die aus Kohlenstoffasermaterial besteht, d a d u r c h   g e k e n n z e i c h n e t ,   daß an der Patientenlagerungsplatte (4) eine Heizstromquelle (19) angeschlossen ist, die durch das Kohlenstoffasermaterial einen Heizstrom zur Beheizung der Lagerfläche (18) schickt.

0145892

<br>

# EUROPÄISCHER RECHERCHENBERICHT

**Europäisches Patentamt**

0145892 Nummer der Anmeldung

EP 84 11 2720

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | BE-A- 688 786 (GENERAL ELECTRIC COMPANY)<br><br>* Seite 3, Zeilen 14-22; Seite 4, Zeilen 2-19; Seite 7, Zeile 18 – Seite 8, Zeile 10; Figuren 1-6 *<br><br>--- | 1 | A 61 B 6/04<br>H 05 B 3/36<br>A 61 G 13/00 |
| Y | EP-A-0 022 919 (MESSERSCHMITT-BÖLKOW-BLOHM GmbH.)<br><br>* Zusammenfassung; Seite 2, Zeilen 12-20; Seite 5, Zeilen 2-9, 20-26; Ansprüche 1,3,5; Figuren 1,3 *<br><br>--- | 1 | |
| Y | US-A-4 146 793 (L. BERGSTROM et al.)<br><br>* Zusammenfassung; Spalte 1, Zeilen 26-33; Spalte 3, Zeilen 34-40; Spalte 4, Zeilen 7-13, 55-60; Figuren 1-5 *<br><br>--- | 1 | **RECHERCHIERTE SACHGEBIETE (Int. Cl.4)**<br><br>A 61 B<br>A 61 G<br>B 32 B<br>H 05 B |
| A | DE-A-2 938 261 (K. DECKER)<br><br>* Seite 1, Anspruch 3; Seite 7, Zeilen 21-28; Figur 1 *<br><br>--- | 1 | |
| A | DE-A-3 146 234 (BAYER AG.)<br><br>* Insgesamt * | 1 | |

Der vorliegende Recherchenbericht wurde für alle Patentansprüche erstellt.

| Recherchenort | Abschlußdatum der Recherche | Prüfer |
|---|---|---|
| DEN HAAG | 14-02-1985 | RIEB D.K. |

EPA Form 1503 03 82